(19) Europäisches Patentamt
European Patent Office
Office européen des brevets

(11) Veröffentlichungsnummer: **0 025 773**
**B1**

(12) **EUROPÄISCHE PATENTSCHRIFT**

(45) Veröffentlichungstag der Patentschrift: **01.08.84**

(21) Anmeldenummer: **80810280.0**

(22) Anmeldetag: **08.09.80**

(51) Int. Cl.³: **C 07 C 149/20,** C 07 D 213/64, A 01 N 29/00, A 01 N 41/10, A 01 N 43/40

# (54) Schwefelhaltige Alkancarbonsäurederivate mit herbizider und den Pflanzenwuchs regulierender Wirkung, deren Herstellung und Verwendung.

(30) Priorität: **13.09.79 CH 8297/79**

(43) Veröffentlichungstag der Anmeldung: **25.03.81 Patentblatt 81/12**

(45) Bekanntmachung des Hinweises auf die Patenterteilung: **01.08.84 Patentblatt 84/31**

(84) Benannte Vertragsstaaten: **BE CH DE FR GB IT LI NL**

(56) Entgegenhaltungen:
**EP - A - 0 000 176**
**EP - A - 0 000 351**
**US - A - 4 093 446**

**CHEMICAL ABSTRACTS, Band 81, Nr. 15, 14. Oktober 1974, Seite 89, Nr. 86762g Columbus, Ohio, U.S.A.**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Szczepanski, Henry, Dr, Waldhuterstrasse 55, CH-4310 Rheinfelden (CH)**
Erfinder: **Rohr, Otto, Dr., Kilbertweg 19, CH-4106 Therwil (CH)**
Erfinder: **Rempfler, Hermann, Dr., Brücklismattstrasse 16, CH-4107 Ettingen (CH)**

**EP 0 025 773 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Diese Erfindung betrifft neue schwefelhaltige Alkancarbonsäurederivate, Verfahren zu ihrer Herstellung, herbizide und den Pflanzenwuchs regulierende Mittel, welche diese Derivate als Wirkstoffe enthalten, sowie die Verwendung derselben.

In der Literatur sind neben zahlreichen auf verschiedenste Weise substituierten Phenoxy-phenoxy-alkancarbonsäuren und Pyridyloxy-phenoxy-alkancarbonsäuren und deren Derivaten auch entsprechende 3- und 4-Phenoxy-phenoxy-alkanole und ihre entsprechenden Phenylthio-Analogen, Mercaptane, Ester und Amine, sowie weitere Derivate bekanntgeworden. (DOS 2 611 695, DOS 2 533 172, USP 4 046 798, USP 4 093 446).

Es wurde nun gefunden, dass gewisse 3-Phenoxy-alkylthio-sulfinyl- und sulfonyl-alkancarbonsäurederivate und 3-Pyridyloxy-phenoxy-alkylthio-sulfinyl und -sulfonylalkancarbonsäurederivate diesen vorbekannten Verbindungen in mancher Weise in ihrer herbiziden und das Pflanzenwachstum regulierenden Wirkung überlegen sind.

Die neuen Wirkstoffe entsprechen der Formel I

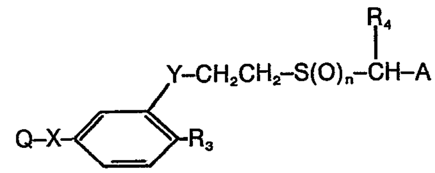

(I)

worin

Q ein Rest

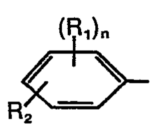

oder

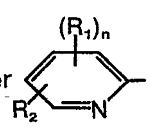

A die Cyanogruppe oder ein Rest $-COB$, B ein Rest $-OR_5$, $-SR_6$, $-NR_7R_8$, $R_1$ ein Halogenatom, n die Zahl 0, 1 oder 2, $R_2$ ein Halogenatom, der Trifluormethyl-, Nitro- oder Cyanorest, $R_3$ ein Halogenatom, der Nitro- oder Cyanorest, Wasserstoff, $R_4$ $C_1$–$C_6$-Alkyl, $C_2$–$C_6$-Alkenyl, $C_2$–$C_6$-Alkinyl, Benzyl oder Phenyl, gegebenenfalls durch Halogen substituiert, $C_2$–$C_6$-Alkoxyalkyl, $C_3$–$C_{12}$-Cycloalkyl,

$R_5$ Wasserstoff oder das Kation einer Base $\frac{1}{m}$ $M^{m\oplus}$, M ein Alkali-, Erdalkali-Kation oder ein Fe, Cu-, Zn-, Mn-, Ni-Kation oder einen Ammonium-Rest

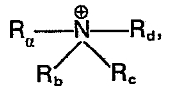

m als ganze Zahl 1, 2 oder 3 die Wertigkeit des Kations berücksichtigt, während $R_a$, $R_b$, $R_c$ und $R_d$ unabhängig voneinander Wasserstoff, Benzyl oder einen gegebenenfalls durch $-OH$, $-NH_2$ oder $C_1$–$C_4$-Alkoxy substituierten $C_1$–$C_4$-Alkylrest bedeuten, weiter bedeutet.

$R_5$ und $R_6$ einen $C_1$–$C_{18}$-Alkyl-Rest, der unsubstituiert oder gegebenenfalls substituiert ist durch Halogen, Nitro, Cyano, $C_1$–$C_8$-Alkoxy, $C_2$–$C_8$-Alkoxyalkoxy, $C_3$–$C_6$-Alkenyloxy, $C_1$–$C_8$-Alkylthio, $C_2$–$C_8$-Alkanoyl, $C_2$–$C_8$-Acyloxy, $C_2$–$C_8$-Alkoxycarbonyl, Carbamoyl; Bis-($C_1$–$C_4$-alkyl)amino, Tris-($C_1$–$C_4$-alkyl)ammonio, $C_3$–$C_8$-Cycloalkyl, $C_3$–$C_8$-Cycloalkenyl, gegebenenfalls auch durch einen unsubstituierten oder seinerseits durch Halogen, $C_1$–$C_4$-Alkyl, $C_1$–$C_4$-Alkoxy ein- oder mehrfach substituierten Phenoxyrest oder 5- bis 6gliedrigen heterocyclischen Rest mit 1 bis 3 Heteroatomen;

einen unsubst. oder ein- bis vierfach durch Halogen oder einmal durch Phenyl oder Methoxycarbonyl subst. $C_3$–$C_{18}$-Alkenyl-Rest;

- einen $C_3$–$C_8$-Alkinyl-Rest;
- einen gegebenenfalls durch Halogen oder $C_1$–$C_4$-Alkyl substituierten $C_3$–$C_{12}$-Cycloalkyl-Rest;
- einen $C_3$–$C_8$-Cycloalkenyl-Rest;
- einen Phenylrest, der unsubstituiert oder durch Halogen, $C_1$–$C_4$-Alkyl, $C_1$–$C_4$-Alkoxy, $C_1$–$C_4$-Alkylthio, $NO_2$, $CF_3$, COOH, CN, OH, $SO_3H$, $NH_2$ oder $-NH(C_1$–$C_4$-Alkyl) oder $-N(C_1$–$C_4$-Alkyl$)_2$ ein- oder mehrfach substituiert ist;
- einen 5 bis 6gliedrigen heterocyclischen Ring mit 1 bis 3 Heteroatomen;

$R_7$ und $R_8$ unabhängig voneinander je Wasserstoff, einen $C_1$–$C_6$-Alkylrest, der durch Sauerstoff unterbrochen oder durch Halogen, Hydroxy oder Cyan substituiert sein kann; einen $C_2$–$C_6$-Alkenyl- oder Alkinylrest, der durch Halogen substituiert sein kann; einen $C_3$–$C_6$-Cycloalkylrest; einen Phenyl- oder Benzylrest, der durch Halogen, $C_1$–$C_4$-Alkyl, Alkoxy oder Alkylthio, Nitro, Cyan oder Trihalogenmethyl substituiert sein kann;

$R_7$ und $R_8$ können zusammen mit dem Stickstoffatom, an das sie gebunden sind, auch einen heterocyclischen Ring bilden;

X und Y je ein Sauerstoff- oder Schwefelatom bedeuten.

Die Alkylreste in dieser Formel können verzweigt oder unverzweigt sein. Die Reste $R_7$ und $R_8$ können Wasserstoff, aliphatische oder aromatische Reste sein oder sie können, zusammen mit dem Stickstoffatom, an das sie gebunden sind, einen vorzugsweise 5–6gliedrigen Heterocyclus bilden, der auch noch ein zweites Heteroatom enthalten kann. Bevorzugt jedoch sind Wasserstoff, niedrige Alkylreste wie auch verzweigte Alkenyl- und Alkinylreste, wie z.B. Allyl, Methallyl 2,2-Dimethylallyl, 2,2-Dimethylpropargyl und 2,2-Diäthylpropargyl.

Als besonders aktiv haben sich diejenigen Verbindungen der Formel I erwiesen, in denen Q ein Rest 2,4-Dichlorphenyl; 3,5-Dichlorpyrid-2-yl, 2-Chlor-4-trifluormethylphenyl oder 3-Chlor-5-trifluormethyl-pyrid-2′-yl, X und Y je Sauerstoff, $R_3$ ein Halogenatom, die Nitro- oder Cyanogruppe, n Null, $R_4$ Wasserstoff oder Methyl und A eine $C_1$–$C_{18}$-Alkoxycarbonyl oder $C_2$–$C_8$-Alkoxyalkoxycarbonylgruppe darstellt.

Die neuen Wirkstoffe der Formel I gemäss vorliegender Erfindung besitzen Herbizidwirkung bei

pre- und post-emergenter Anwendung und können in mono- und dikotylen Kulturen als Unkrautmittel eingesetzt werden. Ferner besitzen sie günstige wachstumsregulierende Effekte (Wuchshemmung). Insbesondere hemmen sie das Wachstum von dicotylen Pflanzen. Beispiele für die nutzbringende Anwendung der erfindungsgemässen Verbindungen sind z.B.

- die Reduktion des vegetativen Wachstums bei Soja und ähnlichen Leguminosen, was zu einer Ertragssteigerung dieser Kultur führt;
- die Hemmung des unerwünschten Wachstums von Geiztrieben bei Tabak, dessen Haupttrieb man geschnitten hat, was der Ausbildung grösserer und schönerer Blätter zugute kommt;
- die Hemmung des Wachstums von Gras und dikotyledonen Pflanzen, wie Obstbäume, Zierbäume, Gebüsche und Hecken, zwecks Einsparung an Schnittarbeit.
- Die Austrocknung und Entblätterung von Pflanzen, z.B. Kartoffeln und Baumwolle, kurz bevor deren Ernte.

Die Aufwandmengen zur Erreichung der gewünschten herbiziden oder wachstumsregulierenden Wirkung liegen zwischen 0,1 und 5 kg Wirkstoff pro Hektare.

Die Herstellung der neuen Verbindungen der Formel I erfolgt nach an sich bekannten Methoden, zum Beispiel gemäss den folgenden Synthesewegen.

Ein Phenol oder Thiophenol der Formel II

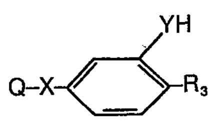

(II)

worin Q, $R_3$, X und Y die unter Formel I gegebene Bedeutung haben, wird in einem polaren organischen Lösungsmittel in Gegenwart der säurebindenden Menge einer Base zuerst mit einem Dihaloäthan der Formel III umgesetzt

$$Hal{-}CH_2{-}CH_2{-}Hal \qquad (III)$$

worin Hal Halogen, vorzugsweise Chlor oder Brom, bedeutet, dann das entstandene Haloäthoxybenzol oder Haloäthoxythiobenzol in einem polaren organischen Lösungsmittel, in Gegenwart der säurebindenden Menge einer Base mit einer Verbindung der Formel IV umsetzt,

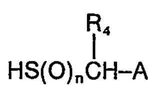

(IV)

worin A, n und $R_4$ die unter Formel I gegebene Bedeutung haben.

Gemäss einem weiteren Weg werden die Verbindungen der Formel I hergestellt, indem man eine Verbindung der Formel V

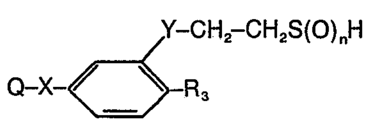

(V)

worin n, Q, $R_3$, X und Y die unter Formel I gegebene Bedeutung haben, in einem polaren organischen Lösungsmittel, in Gegenwart der säurebindenden Menge einer Base, mit einem α-Halogencarbonsäurederivat der Formel VI umgesetzt,

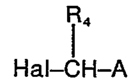

(VI)

worin Hal Halogen, insbesondere Chlor oder Brom, bedeutet und A und $R_4$ die unter Formel I gegebene Bedeutung haben.

Die Verbindungen der Formel I werden auch dadurch hergestellt, dass man ein Phenol oder Thiophenol der Formel II in einem polaren Lösungsmittel in Gegenwart der säurebindenden Menge einer Base mit einem Haloäthylthio-, Sulfinyl- oder Sulfonylalkancarbonsäurederivat der Formel VII umsetzt,

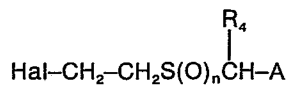

(VII)

worin Hal Halogen, insbesondere Chlor oder Brom, bedeutet und A, n und $R_4$ die unter Formel I gegebene Bedeutung haben.

Schliesslich werden die Sulfinyl- und Sulfonyl-Verbindungen der Formel I auch hergestellt, indem man eine Verbindung der Formel Ia

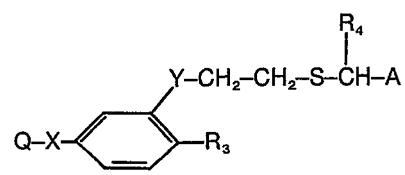

(Ia)

worin A, Q, $R_3$, $R_4$, X und Y die unter Formel I gegebene Bedeutung haben, in einem den Reaktionsteilnehmern gegenüber inerten Lösungsmittels eines Oxidationsmittels oxidiert.

Die genannten Umsetzungen werden in Anwesenheit von gegenüber den Reaktionsteilnehmern inerten Lösungs- oder Verdünnungsmitteln durchgeführt. Bevorzugt sind polare organische Lösungsmittel wie Methyläthylketon, Dimethylformamid, Dimethylsulfoxid usw. Die Reaktionstemperaturen liegen zwischen 0 und 200° und die Reaktionsdauer beträgt je nach Ausgangsstoff, gewählter Umsetzungstemperatur und Lösungsmittel zwischen 1 Stunde und mehreren Tagen. Man arbeitet in der Regel bei Normaldruck. Als Basen (Kondensationsmittel) für die Umsetzungen kommen die üblichen, wie z.B. KOH, $NaOCH_3$, $NaHCO_3$, $K_2CO_3$, Kalium-tert.butylat usw., aber auch organische Basen wie Triäthylamin usw. in Betracht.

Als Oxydationsmittel können für die Umwandlung der Thio- in Sulfinyl- und Sulfonylbrücken beispielsweise Wasserstoffsuperoxyd, Peressigsäure, Perbenzoesäure, 4-Chlorperbenzoesäure, Perlaurinsäure, Natriumperjodat, Jodbenzdichlorid, N-Chlorsuccinimid, N- Bromsuccinimid in Frage. Als Lösungsmittel kommen je nach verwendetem Oxydationsmittel Methylenchlorid, Chloroform, Essigsäure, Wasser usw. in Frage. Ähnliche Oxydationsreaktionen sind beispielsweise in «Organic Compounds of Bivalent Sulfur» Band 2 S. 64 (Chemical Publishing Co. New York 1960) beschrieben.

Die Ausgangsstoffe der Formeln II bis VII sind teilweise bekannt. Noch nicht beschriebene Ausgangsstoffe dieser Formeln lassen sich nach üblichen Verfahren und Techniken leicht herstellen. Phenoxyphenole der Formel II können beispielsweise gemäss den in J. Am. Chem. Soc. 61, 2702 (1939) oder in Chem. Abstract 52 9006b (1958) beschriebenen Methoden hergestellt werden. Die DOS 2 433 066 beschreibt ebenfalls die Herstellung solcher Phenoxy-phenole.

Die Verbindungen der Formel I sind wenig giftig für Warmblüter. Ihre Handhabung und Applikation wirft keine Probleme auf.

Die neuen Wirkstoffe der Formel I sind stabile Verbindungen, welche in üblichen organischen Lösungsmitteln, wie Alkoholen, Äthern, Ketonen, Dimethylformamid, Dimethylsulfoxid usw. löslich sind.

Die Herstellung erfindungsgemässer Mittel erfolgt in an sich bekannter Weise durch inniges Vermischen und Vermahlen von Wirkstoffen der Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden:

Feste Aufarbeitungsformen:
Stäubemittel, Streumittel, Granulate, Umhüllungsgranulate, Imprägnierungsgranulate und Homogengranulate.

In Wasser dispergierbare Wirkstoffkonzentrate:
Spritzpulver (wettable powder), Pasten, Emulsionen, Emulsionenkonzentrate.

Flüssige Aufarbeitungsformen:
Lösungen, Dispersionen.

Die Wirkstoffkonzentrationen betragen in den erfindungsgemässen Mitteln 1 bis 80 Gewichtsprozent und können gegebenenfalls bei der Anwendung auch in geringen Konzentrationen wie etwa 0,05 bis 1% vorliegen.

In den beschriebenen erfindungsgemässen Mitteln lassen sich andere biozide Wirkstoffe beimischen.

In den nachfolgenden Beispielen wird diese Erfindung weiter veranschaulicht. Die Temperaturen sind darin in Celsiusgraden gegeben, Druckangaben in Millibar, Teile und Prozentangaben beziehen sich auf das Gewicht.

Beispiel 1:
Herstellung eines Wirkstoffes
3-(2'-Chlor-4'-trifluormethylphenoxy)-6-chlorphenoxy-äthylthioessigsäuremethylester:

20,7 g Kaliumcarbonat, 19,2 g (0,05 Mol) 3-(2'-Chlor-4'-trifluormethylphenoxy)-6-chlorphenoxychloräthan und 5,3 g (0,05 Mol) Thioglykolsäuremethylester werden bei Raumtemperatur in 80 ml Dimethylsulfoxid zusammengegeben. Bei der exothermen Reaktion steigt die Temperatur auf ca. 42 °C. Dann wird über Nacht bei Raumtemperatur weitergerührt. Am nächsten Tag wird mit 300 ml Äther verdünnt und zweimal mit je 400 ml 20%iger $K_2CO_3$-Lösung gewaschen. Die organische Phase wird über Magnesiumsulfat getrocknet und eingedampft. Man erhält so 17,8 g hellgelbes Öl, das einen Brechungsindex von $n^{21}_D$ 1.5487 aufweist.

In analoger Weise zu diesem Beispiel wurden folgende Verbindungen hergestellt:

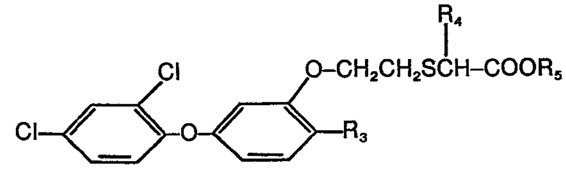

| $R_3$ | $R_4$ | $R_5$ | physikalische Daten |
|---|---|---|---|
| Cl | H | $C_3H_7$iso | $n^{22}_D$ 1.5688 |
| Cl | H | $C_2H_5$ | $n^{22}_D$ 1.5778 |
| Cl | H | $CH_3$ | $n^{25}_D$ 1.5813 |
| Cl | H | $C_2H_4OCH_3$ | $n^{23}_D$ 1.5644 |
| Cl | H | $C_4H_9$n | $n^{23}_D$ 1.5582 |
| Cl | H | $C_8H_{17}$i | $n^{23}_D$ 1.5450 |
| Cl | $CH_3$ | $C_2H_5$ | $n^{22}_D$ 1.5529 |
| Br | $CH_3$ | $CH_3$ | |
| Br | $CH_3$ | $C_3H_7$iso | |
| CN | $CH_3$ | $CH_3$ | |
| CN | $CH_3$ | $CH_3$ | |
| CN | H | $CH_3$ | |

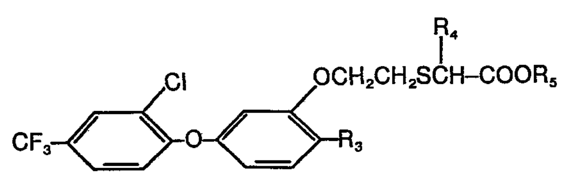

| $R_3$ | $R_4$ | $R_5$ | physikalische Daten |
|---|---|---|---|
| Cl | $CH_3$ | $CH_3$ | $n^{22}_D$ 1.5430 |
| Cl | H | $C_2H_5$ | $n^{22}_D$ 1.5438 |
| Cl | $CH_3$ | $C_2H_5$ | $n^{22}_D$ 1.5381 |
| Cl | H | $CH_3$ | $n^{22}_D$ 1.5487 |
| Cl | H | $C_4H_9$n | $n^{22}_D$ 1.5325 |
| Cl | H | $C_8H_{17}$ iso | $n^{23}_D$ 1.5189 |
| Cl | H | $C_2H_4OCH_3$ | $n^{21}_D$ 1.5351 |
| $NO_2$ | $CH_3$ | $CH_3$ | $n^{23}_D$ 1.5435 |
| $NO_2$ | H | $C_4H_9$n | $n^{22}_D$ 1.5378 |
| $NO_2$ | H | $C_2H_5$ | $n^{22}_D$ 1.5460 |
| $NO_2$ | $CH_3$ | $C_2H_5$ | $n^{23}_D$ 1.5340 |
| $NO_2$ | H | $C_3H_7$ iso | $n^{27}_D$ 1.5621 |
| $NO_2$ | H | $C_2H_4OCH_3$ | $n^{27}_D$ 1.5430 |

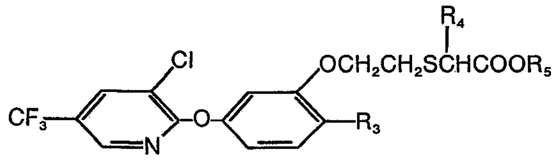

| $R_3$ | $R_4$ | $R_5$ | physikalische Daten |
|---|---|---|---|
| Cl | $CH_3$ | $CH_3$ | |
| Cl | H | $CH_3$ | |
| CN | $CH_3$ | $CH_3$ | |
| CN | H | $C_2H_5$ | |
| $NO_2$ | $CH_3$ | $CH_3$ | |
| $NO_2$ | H | $CH_3$ | |

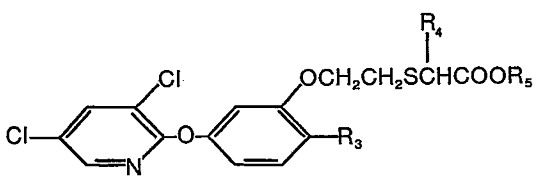

| | | | |
|---|---|---|---|
| Cl | $CH_3$ | $CH_3$ | $n_D^{30}$ 1.5790 |
| Cl | H | $CH_3$ | $N_D^{30}$ 1.5988 |
| $NO_2$ | $CH_3$ | $CH_3$ | |
| $NO_2$ | H | $CH_3$ | |
| CN | $CH_3$ | $CH_3$ | |
| CN | H | $CH_3$ | |

Beispiel 2:
Herstellung einiger Aufbereitungsformen

Granulat

Zur Herstellung eines 5%igen Granulates werden die folgenden Stoffe verwendet:

5 Teile 3-(2',4'-Dichlorphenoxy)-6-chlorphenoxy-alkylthio-essigsäuremethylester,
0,25 Teile epoxidiertes Pflanzenöl
0,25 Teile Cetylpolyglykoläther,
3,50 Teile Polyäthylenglykol,
91 Teile Kaolin (Korngrösse: 0,3–0,8 mm).

Die Aktivsubstanz wird mit dem Pflanzenöl vermischt und in 6 Teilen Aceton gelöst, hierauf wird Polyäthylenglykol und Cetylpolyglykoläther zugesetzt. Die so erhaltene Lösung wird auf Kaolin aufgesprüht und anschliessend im Vacuum verdampft.

Spritzpulver

Zur Herstellung eines a) 70%igen und b) 10%igen Spritzpulvers werden folgende Bestandteile verwendet:

a) 70 Teile 3-(4'-Trifluormethyl-phenoxy)-6-chlorphenoxy]-äthylthio-essigsäuremethylester,
5 Teile Natriumdibutylnaphthylsulfonat,
3 Teile Naphthalinsulfonsäure-Phenolsulfonsäure-Formaldehyd-Kondensat 3 : 2 : 1,
10 Teile Kaolin
12 Teile Champagne-Kreide;

b) 10 Teile des obigen Wirkstoffes
3 Teile Gemisch der Natriumsalze von gesättigten Fettalkoholsulfaten,
5 Teile Naphthalinsulfonsäuren-Formaldehyd Kondensat,
82 Teile Kaolin.

Der Wirkstoff wird auf die entsprechenden Trägerstoffe Kaolin und Kreide) aufgezogen und anschliessend mit den übrigen Bestandteilen vermischt und vermahlen. Man erhält Spritzpulver von vorzüglicher Benetzbarkeit und Schwebefähigkeit. Aus solchen Spritzpulvern können durch Verdünnen mit Wasser Suspensionen von 0,1–80% Wirkstoff erhalten werden, die sich zur Unkrautbekämpfung in Pflanzenkulturen eignen.

Paste

Zur Herstellung einer 45%igen Paste werden folgende Stoffe verwendet:

45 Teile 3-(4'-Trifluormethylphenoxy)-6-chlorphenoxy-äthylthio-essigsäure-methylester oder eines anderen der genannten Wirkstoffe der Formel I,
5 Teile Natriumaluminiumsilikat,
14 Teile Cetylpolyglykoläther mit 8 Mol Äthylenoxid,
1 Teil Oeylpolyglykoläther mit 5 Mol Äthylenoxyd,
2 Teile Spindelöl,
10 Teile Polyäthylenglykol,
23 Teile Wasser.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat

Zur Herstellung eines 25%igen Emulsionskonzentrates werden

25 Teile 2-[3-(3',5'-Dichlorpyridyl-2'-oxy)-6-phenoxy]-äthylthio-essigsäuremethylester
5 Teile einer Mischung von Nonylphenolpolyoxyäthylen oder Calciumdodecylbenzolsulfat,
15 Teile Cyclohexanon,
55 Teile Xylol

miteinander vermischt. Dieses Konzentrat kann mit Wasser zu Emulsionen auf geeignete Konzentrationen von z.B. 0,1 bis 10% verdünnt werden.

Beispiel 3:
Nachweis der biologischen Wirkung

Pre-emergente Herbizid-Wirkung (Keimhemmung)

Im Gewächshaus wird unmittelbar nach der Einsaat der Versuchspflanzen in Saatschalen die Erdoberfläche mit einer wässerigen Dispersion der Wirkstoffe, erhalten aus einem 25%igen Emulsionskonzentrat resp. aus einem 25%igen Spritzpulver mit Wirkstoffen, die wegen ungenügender Löslichkeit nicht als Emulsionskonzentrate hergestellt werden können, behandelt. Es wurden vier verschiedene Konzentrationsreihen angewendet,

entsprechend 4, 2, 1 und 0,5 kg Wirksubstanz pro Hektar. Die Saatschalen werden im Gewächshaus bei 22–25 °C und 50–70% rel. Luftfeuchtigkeit gehalten und der Versuch nach 3 Wochen ausgewertet. Als Versuchspflanzen dienen:

| | |
|---|---|
| Hordeum (Gerste) | setaria italica |
| triticum (Weizen) | echinochloa crus galli |
| zea (Mais) | beta vulgaris |
| sorghum hybr. (Hirse) | sida spinosa |
| oryza (Reis) | sesbania exaltata |
| glycine (Soja) | amaranthus retroflexus |
| gossypium (Baumwolle) | sinapis alba |
| avena fatua | ipomoea purpurea |
| lolium perenne | galium aparine |
| alopecurus myosuroides | pastinaca sativa |
| bromus tectorum | rumex sp. |
| cyperus esculentus | chrysanthemum leucum. |
| rottboellia exaltata | abutilon sp. |
| digitaria sanguinalis | solanum nigrum |

Post-emergente Herbizid-Wirkung (Kontaktherbizid)

Eine grössere Anzahl (mindestens 7) Unkräuter und Kulturpflanzen, sowohl monocotyle wie dicotyle, wurde nach dem Auflaufen (im 4- bis 6-Blattstadium) mit einer wässerigen Wirkstoffdispersion in Dosierungen von 0,06; 0,125; 0,25; 0,5; 1; 2 und 4 kg Wirksubstanz pro Hektar auf die Pflanzungen gespritzt und diese bei 24–26 °C und 45–60% rel. Luftfeuchtigkeit gehalten. 15 Tage nach Behandlung wird der Versuch ausgewertet.

Hemmung des vegetativen Wachstums in Sojapflanzen

In einem Sojabohnenfeld wurden Parzellen von je 30 auf 8 Fuss mit wässrigen Lösungen der Wirkstoffe zu einem Zeitpunkt bespritzt, wo sich die Sojapflanzen im 9–10 Blatt-Stadium befanden. Unbehandelte Parzellen dienten als Kontrolle. Im Erntezeitpunkt, 3½ Monate nach dem Applikationszeitpunkt, wurde die mittlere Wuchshöhe der Pflanzen in jeder Parzelle sowie die Erträge bestimmt und mit denen der unbehandelten Kontrollpflanzen verglichen.

Defoliation und Dessikation in Baumwolle

In einem Baumwoll-Feld wurden 2 Wochen vor dem erwarteten Erntezeitpunkt Parzellen von je 2 Reihen auf 20 Fuss mit wässrigen Zubereitungen der Wirkstoffe bespritzt. Unbehandelte Parzellen dienten als Kontrollen. Am 3., 7 und 14. Tag nach der Applikation wurde der Blattfall und die Dessikationswirkung an den verschiedenen Parzellen bestimmt und mit dem Zustand der Kontrollpflanzen verglichen.

In diesen Versuchen waren alle genannten Verbindungen aktiv. Mit folgenden Verbindungen wurden die besten Resultate erreicht:

2-[3-(2'-Chlor-4'-trifluormethylphenoxy)-6-chlorphenoxy-äthylthio]-propionsäuremethylester

2-(2'-Chlor-4'-trifluormethylphenoxy)-6-chlorphenoxy-äthylthio-essigsäuremethylester

3-(2'-Chlor-4'-trifluormethylphenoxy-6-nitrophenoxy-äthylthio-essigsäureäthylester

2-[3-(2'-Chlor-4'-trifluormethylphenoxy)-6-nitrophenoxy-äthylthio]-propionsäuremethylester

2-[3-(2'-Chlor-4'-trifluormethylphenoxy)-6-nitrophenoxy-äthylthio]-propionsäureäthylester.

**Patentansprüche**

1. Schwefelhaltige Alkancarbonsäurederivate der Formel I

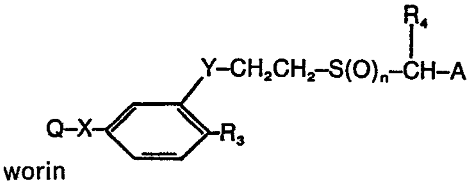

(I)

worin

Q ein Rest

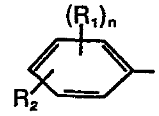

oder

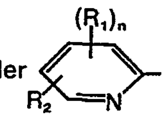

A die Cyanogruppe oder ein Rest –COB, B ein Rest $-OR_5$, $-SR_6$, $-NR_7R_8$, $R_1$ ein Halogenatom, n die Zahl 0, 1 oder 2, $R_2$ ein Halogenatom, der Trifluormethyl-, Nitro-, oder Cyanorest, $R_3$ ein Halogenatom, der Nitro- oder Cyanorest, Wasserstoff, $R_4$ $C_1$–$C_6$-Alkyl, $C_2$–$C_6$-Alkenyl, $C_2$–$C_6$-Alkinyl, Benzyl oder Phenyl, gegebenenfalls durch Halogen substituiert, $C_2$–$C_6$-Alkoxyalkyl, $C_3$–$C_{12}$-Cycloalkyl, $R_5$ Wasserstoff oder das Kation einer Base $\frac{1}{m} M^{m\oplus}$, M ein Alkali-, Erdalkali-Kation oder ein Fe-, Cu-, Zn-, Mn-, Ni-Kation oder einen Ammonium-Rest

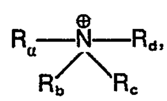

m als ganze Zahl 1, 2 oder 3 die Wertigkeit des Kations berücksichtigt, während $R_a$, $R_b$, $R_c$ und $R_d$ unabhängig voneinander Wasserstoff, Benzyl oder einen gegebenenfalls durch –OH, $-NH_2$ oder $C_1$–$C_4$-Alkoxy substituierten $C_1$–$C_4$-Alkylrest bedeuten, weiter bedeutet $R_5$ und $R_6$ einen $C_1$–$C_{18}$-Alkyl-Rest, der unsubstituiert oder gegebenenfalls substituiert ist durch Halogen, Nitro, Cyano, $C_1$–$C_8$-Alkoxy, $C_2$–$C_8$-Alkoxyalkoxy, $C_3$–$C_6$-Alkenyloxy, $C_2$–$C_8$-Alkoxycarbonyl, Carbamoyl; Bis-($C_1$–$C_4$ alkyl)amino, Tris-($C_1$–$C_4$ alkyl)ammonio, $C_3$–$C_8$-Cycloalkyl, $C_3$–$C_8$-Cycloalkenyl, gegebenenfalls auch durch einen unsubstituierten oder seinerseits durch Halogen, $C_1$–$C_4$-Alkyl, $C_1$–$C_4$-Alkoxy ein- oder mehrfach substituierten Phenoxyrest oder 5–6gliedrigen heterocyclischen Rest mit 1 bis 3 Heteroatomen;

- einen unsubst. oder ein- bis vierfach durch Halogen oder einmal durch Phenyl oder Methoxycarbonyl subst. $C_3$–$C_{18}$-Alkenylrest;
- einen $C_3$–$C_8$-Alkinyl-Rest;
- einen gegebenenfalls durch Halogen oder $C_1$–$C_4$-Alkyl substituierten $C_3$–$C_{12}$-Cycloalkyl-Rest;

- einen $C_3$–$C_8$-Cycloalkenyl-Rest;
- einen Phenylrest, der unsubstituiert oder durch Halogen, $C_1$–$C_4$-Alkyl, $C_1$–$C_4$-Alkoxy, $C_1$–$C_4$-Alkylthio, $NO_2$, $CF_3$, COOH, CN, OH, $SO_3H$, $NH_2$ oder $-NH(C_1$–$C_4$-Alkyl) oder $-N(C_1$–$C_4$-Alkyl$)_2$ ein- oder mehrfach substituiert ist;
- einen 5- bis 6gliedrigen heterocyclischen Ring mit 1 bis 3 Heteroatomen;

$R_7$ und $R_8$ unabhängig voneinander je Wasserstoff, einen $C_1$–$C_6$-Alkylrest, der durch Sauerstoff unterbrochen oder durch Halogen, Hydroxy oder Cyan substituiert sein kann; einen $C_2$–$C_6$-Alkenyl- oder Alkinylrest, der durch Halogen substituiert sein kann; einen $C_3$–$C_6$-Cycloalkylrest; einen Phenyl- oder Benzylrest, der durch Halogen, $C_1$–$C_4$-Alkyl, Alkoxy oder Alkylthio, Nitro, Cyan oder Trihalogenmethyl substituiert sein kann,

$R_7$ und $R_8$ können zusammen mit dem Stickstoffatom, an das sie gebunden sind, auch einen heterocyclischen Ring bilden,

X, und Y je ein Sauerstoff- oder Schwefelatom bedeuten.

2. Die Verbindungen gemäss Patentanspruch 1 der Formel I, in denen Q den Rest 2,4-Dichlorphenyl, 3,5-Dichlorpyrid-2-yl, 2-Chlor-4-trifluormethylphenyl oder 3-Chlor-5-trifluormethylpyrid-2-yl, X und Y je Sauerstoff, n Null, $R_3$ ein Halogenatom, die Nitro- oder Cyanogruppe, $R_4$ Wasserstoff oder Methyl und A eine $C_1$–$C_{18}$ Alkoxycarbonyl- oder $C_2$–$C_9$-Alkoxyalkoxycarbonylgruppe bedeutet.

3. Die Verbindungen gemäss Patentanspruch 1 der Formel

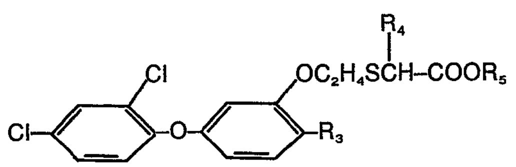

in denen $R_3$ ein Halogenatom, die Nitro- oder Cyanogruppe, $R_4$ Wasserstoff oder Methyl, $R_5$ $C_1$–$C_{18}$-Alkyl oder $C_2$–$C_9$-Alkoxyalkyl bedeuten.

4. Die Verbindungen gemäss Patentanspruch 1 der Formel

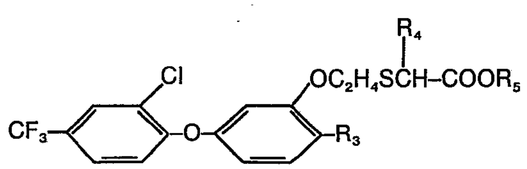

worin $R_3$ ein Halogenatom, die Nitro- oder Cyanogruppe, $R_4$ Wasserstoff oder Methyl $R_5$ $C_1$–$C_{18}$-Alkyl oder $C_2$–$C_9$-Alkoxyalkyl bedeuten.

5. Die Verbindungen gemäss Patentanspruch 1 der Formel

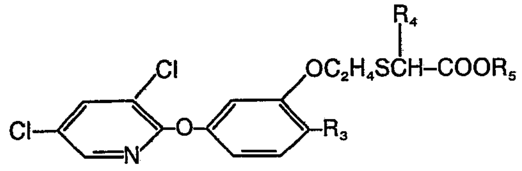

worin $R_3$ ein Halogenatom, die Nitro- oder Cyanogruppe, $R_4$ Wasserstoff oder Methyl, $R_5$ $C_1$–$C_{18}$-Alkyl oder $C_2$–$C_9$-Alkoxyalkyl bedeuten.

6. Die Verbindungen gemäss Patentanspruch 1 der Formel

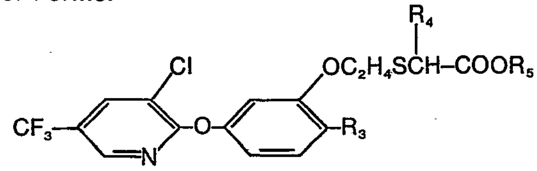

worin $R_3$ ein Halogenatom, die Nitro- oder Cyanogruppe, $R_4$ Wasserstoff oder Methyl, $R_5$ $C_1$–$C_{18}$ Alkyl oder $C_2$–$C_9$ Alkoxyalkyl bedeuten.

7. 2-[3-(2′-Chlor-4′-trifluormethylphenoxy)-6-chlorphenoxy-äthylthio]-propionsäuremethylester, gemäss Patentanspruch 1.

8. 3-(2′-Chlor-4′-trifluormethylphenoxy)-6-chlorphenoxy-äthylthio-essigsäuremethylester, gemäss Patentanspruch 1.

9. 3-(2′-Chlor-4′-trifluormethylphenoxy)-6-nitrophenoxy-äthylthio-essigsäureäthylester, gemäss Patentanspruch 1.

10. 2-[3-(2′-Chlor-4′-trifluormethylphenoxy)-6-nitrophenoxy-äthylthio]-propionsäuremethylester, gemäss Patentanspruch 1.

11. 2-[3-(2′-Chlor-4′-trifluormethylphenoxy)-6-nitrophenoxy-äthylthio-]-propionsäureäthylester, gemäss Patentanspruch 1.

12. Verfahren zur Herstellung der Verbindungen der Formel I, Anspruch 1, dadurch gekennzeichnet, dass man ein Phenol oder Thiophenol der Formel II

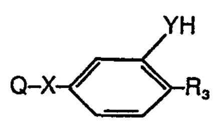

(II)

worin Q, $R_3$, X und Y die unter Formel I, Anspruch I gegebene Bedeutung haben, in einem polaren organischen Lösungsmittel in Gegenwart der säurebindenden Menge einer Base zuerst mit einem 1,2-Dihaloäthan der Formel III umsetzt.

$$Hal-CH_2-CH_2-Hal \quad (III)$$

worin Hal Halogen bedeutet, dann das entstandene Haloäthoxybenzol resp. Haloäthylthiobenzol in einem polaren, organischen Lösungsmittel, in Gegenwart der säurebindenden Menge einer Base mit einer Verbindung der Formel IV umsetzt

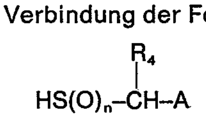

(IV)

worin A, n und $R_4$ die unter Formel I, Anspruch 1 gegebene Bedeutung haben.

13. Verfahren zur Herstellung der Verbindungen der Formel I, dadurch gekennzeichnet, dass man eine Verbindung der Formel V

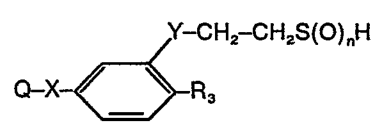

(V)

worin n, Q, $R_3$ und Y die unter Formel I, Anspruch 1, gegebene Bedeutung haben, in einem polaren organischen Lösungsmittel in Gegenwart der säurebindenden Menge einer Base, mit einem α-Halogencarbonsäurederivat der Formel VI umsetzt,

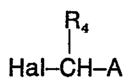

worin Hal Halogen bedeutet und A und $R_4$ die unter Formel I, Anspruch 1, gegebene Bedeutung haben.

14. Verfahren zur Herstellung der Verbindungen der Formel I, Anspruch 1, dadurch gekennzeichnet, dass man ein Phenol oder Thiophenol der Formel II

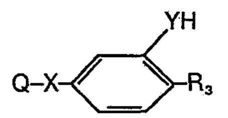

(II)

worin Q, $R_3$, X und Y die unter Formel I, Anspruch 1, gegebene Bedeutung haben, in einem polaren Lösungsmittel, in Gegenwart der säurebindenden Menge einer Base mit einem Haloäthyl-thio-, -sulfinyl- oder -sulfonyl-alkancarbonsäurederivat der Formel VII umsetzt

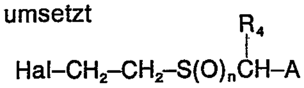

(VIII)

worin Hal Halogen bedeutet und A, n und $R_4$ die unter Formel I, Anspruch 1, gegebene Bedeutung haben.

15. Verfahren zur Herstellung der Sulfinyl- und Sulfonyl-Verbindungen der Formel I, dadurch gekennzeichnet, dass man eine Verbindung der Formel Ia

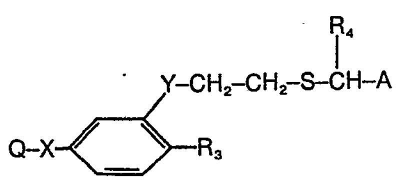

(Ia)

worin A, Q, $R_3$, $R_4$, X und Y die unter Formel I, Anspruch 1, gegebene Bedeutung haben, in einem inerten Lösungsmittel mittels eines Oxydationsmittels oxidiert.

16. Herbizides und den Pflanzenwuchs regulierendes Mittel, dadurch gekennzeichnet, dass es als wirksame Komponente mindestens eine Verbindung der Formel I, Anspruch 1, enthält.

17. Die Verwendung der Verbindungen der Formel I, Anspruch 1 oder sie enthaltender Mittel zur Bekämpfung von Unkräutern.

18. Die Verwendung der Verbindungen der Formel I, Anspruch 1 oder sie enthaltender Mittel zur Hemmung des Pflanzenwachstums.

19. Die Verwendung der Verbindungen der Formel I, Anspruch 1 oder sie enthaltender Mittel zur Dessikation von Kulturpflanzen kurz bevor deren Ernte.

**Claims**

1. A sulfur-containing alkanecarboxylic acid derivative of the formula I

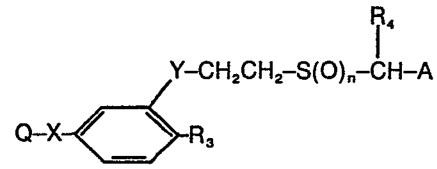

(I)

wherein Q is a radical

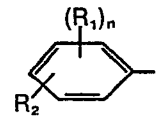

or

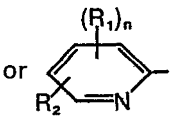

A is the cyano group or a –COB radical, B is a $-OR_5$, $-SR_6$, $-NR_7R_8$ radical, $R_1$ is a halogen atom, n is 0, 1 or 2, $R_2$ is a halogen atom, the trifluoromethyl, nitro or cyano group, $R_3$ is a halogen atom or the nitro or cyano group, $R_4$ is $C_1$–$C_6$-alkyl, $C_2$–$C_6$-alkenyl, $C_2$–$C_6$-alkynyl; benzyl or phenyl, each unsubstituted or substituted by halogen; or is $C_2$–$C_6$-alkoxyalkyl or $C_3$–$C_{12}$-cycloalkyl, $R_5$ is hydrogen or the cation of a base $\frac{1}{m} M^{m\oplus}$, wherein M is an alkaly metal cation or an alkaline earth metal cation or an iron, copper, zinc, manganese or nickel cation or an ammonium radical

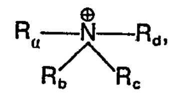

m as an integer 1, 2 or 3 corresponds to the valency of the cation, and each of $R_a$, $R_b$, $R_c$ and $R_d$ independently is hydrogen, benzyl, or a $C_1$–$C_4$ alkyl radical which is unsubstituted or substituted by –OH, $-NH_2$ or $C_1$–$C_4$-alkoxy; each of $R_5$ and $R_6$ is also a $C_1$–$C_{18}$-alkyl radical which is unsubstituted or substituted by halogen, nitro, cyano, $C_1$–$C_8$-alkoxy, $C_2$–$C_8$-alkoxyalkoxy, $C_3$–$C_6$-alkenyloxy, $C_1$–$C_8$-alkylthio, $C_2$–$C_8$-alkanoyl, $C_2$–$C_8$-acyloxy, $C_2$–$C_8$-alkoxycarbonyl, carbamoyl, bis($C_1$–$C_4$-alkyl)amino, tris($C_1$–$C_4$-alkyl)ammonio, $C_3$–$C_8$ cycloalkyl, $C_3$–$C_8$ cycloalkenyl, or also by a phenoxy or 5- or 6-membered heterocyclic radical containing 1 to 3 hetero-atoms, each of which is unsubstituted or is itself mono- or polysubstituted by halogen, $C_1$–$C_4$-alkyl or $C_1$–$C_4$-alkoxy; or is a $C_3$–$C_{18}$-alkenyl radical which is unsubstituted or mono- to tetrasubstituted by halogen or monosubstituted by phenyl or methoxycarbonyl; a $C_3$–$C_8$-alkynyl radical; a $C_3$–$C_{12}$-cycloalkyl radical which is unsubstituted or substituted by halogen or $C_1$–$C_4$-alkyl; a $C_3$–$C_8$-cycloalkylene radical; a phenyl radical which is unsubstituted or mono- or polysubstituted by halogen, $C_1$–$C_4$-alkyl, $C_1$–$C_4$-alkoxy, $C_1$–$C_4$-alkylthio, $NO_2$, $CF_3$, COOH, CN, OH, $SO_3H$, $NH_2$ or $-NH(C_1$–$C_4$-alkyl) or $-N(C_1$–$C_4$-alkyl$)_2$; a 5- to 6-membered heterocyclic ring containing 1 to 3 hetero atoms; each of $R_7$ and $R_8$ independently is hydrogen, a $C_1$–$C_6$-alkyl radical which can be interrupted by oxygen or substituted by halogen, hydroxy or cyano; a $C_2$–$C_6$-alkenyl or $C_2$–$C_6$-

alkynyl radical which can be substituted by halogen; a $C_3$–$C_8$-cycloalkyl radical; a phenyl or benzyl radical which can be substituted by halogen, $C_1$–$C_4$-alkyl, alkoxy or alkylthio, nitro, cyano or trihalomethyl; or $R_7$ and $R_8$, together with the nitrogen atom to which they are attached, can also form a heterocyclic ring; and each of X and Y is an oxygen or a sulfur atom.

2. A compound according to claim 1 of formula I, wherein Q is a 2,4-dichlorophenyl, 3,5-dichloropyrid-2-yl, 2-chloro-4-trifluoromethylphenyl or 3-chloro-5-trifluoromethylpyrid-2-yl radical, each of X and Y is oxygen, $R_3$ is a halogen atom, the nitro or cyano group, n is 0, $R_4$ is hydrogen or methyl, and A is a $C_1$–$C_{18}$-alkoxycarbonyl or $C_2$–$C_9$-alkoxyalkoxycarbonyl group.

3. A compound according to claim 1 of the formula

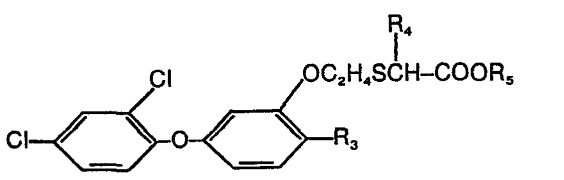

wherein $R_3$ is a halogen atom, the nitro or cyano group, $R_4$ is hydrogen or methyl, $R_5$ is $C_1$–$C_{18}$-alkyl or $C_2$–$C_9$-alkoxyalkyl.

4. A compound according to claim 1 of the formula

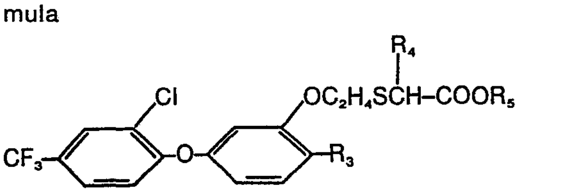

wherein $R_3$ is a halogen atom, the nitro or cyano group, $R_4$ is hydrogen or methyl, $R_5$ is $C_1$–$C_{18}$-alkyl or $C_2$–$C_9$-alkoxyalkyl.

5. A compound according to claim 1 of the formula

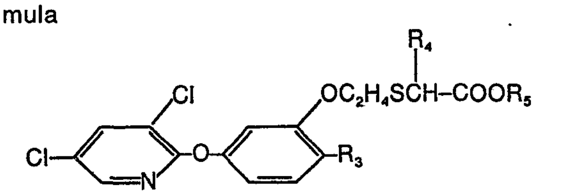

wherein $R_3$ is a halogen atom, the nitro or cyano group, $R_4$ is hydrogen or methyl, $R_5$ is $C_1$–$C_{18}$-alkyl or $C_2$–$C_9$-alkoxyalkyl.

6. A compound according to claim 1 of the formula

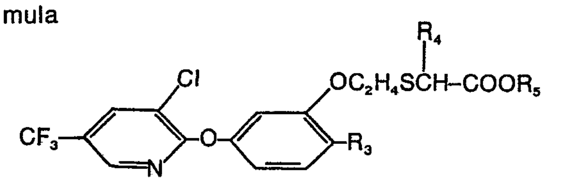

wherein $R_3$ is a halogen atom, the nitro or cyano group, $R_4$ is hydrogen or methyl, $R_5$ is $C_1$–$C_{18}$-alkyl or $C_2$–$C_9$-alkoxyalkyl.

7. Methyl 3-[3-(2′-chloro-4′-trifluoromethylphenoxy)-6-chlorophenoxyethylthio]propionate accord-ing to claim 1.

8. Methyl 3-(2′-chloro-4′-trifluoromethylphenoxy)-6-chlorophenoxyethylthioacetate according to claim 1.

9. Methyl 3-(2′-chloro-4′-trifluoromethylphenoxy)-6-nitrophenoxyethylthioacetate according to claim 1.

10. Methyl 2-[3-(2′-chloro-4′-trifluoromethylphenoxy)-6-nitrophenoxyethylthio]propionate according to claim 1.

11. Ethyl 2-[3-(2′-chloro-4′-trifluoromethylphenoxy)-6-nitrophenoxyethylthio]propionate according to claim 1.

12. A process for the production of a compound of the formula I according to claim 1, which process comprises reacting a phenol or thiophenol of the formula II

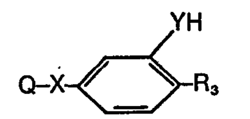 (II)

wherein Q, $R_3$, X and Y are as defined for formula I in claim 1, in a polar organic solvent and in the presence of an acid acceptor, firstly with a 1,2-dihaloethane of the formula III

$$Hal–CH_2–CH_2–Hal \quad (III)$$

wherein Hal is halogen, then reacting the haloethoxybenzene or haloethylthiobenzene thereby obtained, in a polar organic solvent and in the presence of an acid acceptor, with a compound of the formula IV

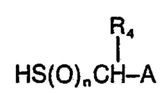 (IV)

wherein A, n and $R_4$ are as defined for formula I in claim 1.

13. A process for the production of a compound of the formula I, which process comprises reacting a compound of the formula V

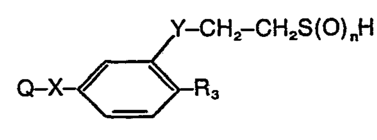 (V)

wherein n, Q, $R_3$ and Y are as defined for formula I in claim 1, in a polar organic solvent and in the presence of an acid acceptor, with an α-halocarboxylic acid derivative of the formula VI

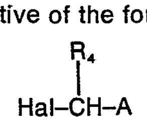 (VI)

wherein Hal is halogen and A and $R_4$ are as defined for formula I in claim 1.

14. A process for the production of a compound of the formula I, which process comprises reacting a phenol or thiophenol of the formula II

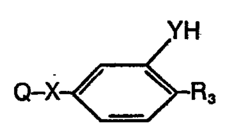 (II)

wherein Q, $R_3$, X and Y are as defined for formula I in claim 1, in a polar organic solvent and in the presence of an acid acceptor, with a haloethylthio-, -sulfinyl- or -sulfonylalkanecarboxylic acid derivative of the formula VII

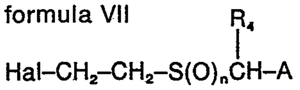
(VII)

wherein Hal is halogen and A, n and $R_4$ are as defined for formula I in claim 1.

15. A process for the production of a sulfinyl or sulfonyl compound of the formula I, which process comprises oxidising a compound of the formula Ia

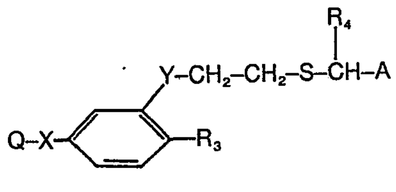
(Ia)

wherein A, Q, $R_3$, $R_4$, X and Y are as defined for formula I in claim 1, in an inert solvent, with an oxidising agent.

16. A herbicidal and plant growth-regulating composition which contains, as active component, at least one compound of the formula I according to claim 1.

17. A method of controlling weeds at a locus which comprises applying to said locus a herbicidally effective amount of a compound of the formula I according to claim 1 or of a composition containing such a compound.

18. A method of inhibiting plant growth which comprises applying to plants an effective amount of a compound of the formula I according to claim 1 or of a composition containing such a compound.

19. A method of desiccating and defoliating cultivated plants shortly before harvesting which comprises applying to said plants a compound of the formula I according to claim 1 or of a composition containing such a compound.

**Revendications**

1. Dérivés soufrés d'acides alcane-carboxyliques de formule I

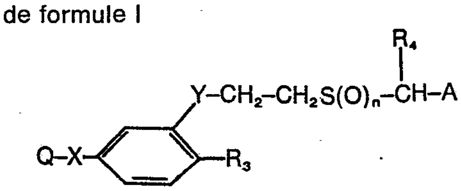
(I)

dans laquelle

Q représente un reste

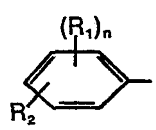
ou
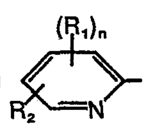

A représente le groupe cyano ou un reste –COB, B représente un reste $-OR_5$, $-SR_6$, $-NR_7R_8$, $R_1$ représente un atome d'halogène, n est égal à 0, 1 ou 2, $R_2$ représente un atome d'halogène, un groupe trifluorométhyle, nitro ou cyano, $R_3$ représente un atome d'halogène, un groupe nitro ou cyano, l'hydrogène, $R_4$ représente un groupe alkyle en $C_1$–$C_6$, alcényle en $C_2$–$C_6$, alcynyle en $C_2$–$C_6$, benzyle ou phényle, éventuellement substitué par des halogènes, un groupe alcoxyalkyle en $C_2$–$C_6$, cycloalkyle en $C_3$–$C_{12}$, $R_5$ représente l'hydrogène ou le cation d'une base $\frac{1}{m} M^{m\oplus}$, M représente un cation de métal alcalin, de métal alcalino-terreux ou un cation de Fe, de Cu, de Zn, de Mn, de Ni ou un reste ammonium

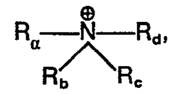

m, qui est un nombre entier égal à 1, 2 ou 3, correspond à la valence du cation, $R_a$, $R_b$, $R_c$ et $R_d$ représentent chacun, indépendamment les uns des autres, l'hydrogène, un groupe benzyle ou un groupe alkyle en $C_1$–$C_4$ éventuellement substitué par des groupes OH, $NH_2$ ou alcoxy en $C_1$–$C_4$, $R_5$ et $R_6$ représentent un groupe alkyle en $C_1$–$C_{18}$ non substitué ou éventuellement substitué par des halogènes, des groupes nitro, cyano, alcoxy en $C_1$–$C_8$, alcoxyalcoxy en $C_2$–$C_8$, alcényloxy en $C_3$–$C_6$, alkylthio en $C_1$–$C_8$, alcanoyle en $C_2$–$C_8$, acyloxy en $C_2$–$C_8$, alcoxycarbonyle en $C_2$–$C_8$, carbamoyle; bis-(alkyle en $C_1$–$C_4$)-amino, tris-(alkyle en $C_1$–$C_4$)-ammonio, cycloalkyle en $C_3$–$C_8$, cycloalcényle en $C_3$–$C_8$, le cas échéant également par un reste phénoxy ou un reste hétérocyclique à 5 ou 6 chaînons contenant de 1 à 3 hétéroatomes, non substitué ou lui-même substitué une ou plusieurs fois par des halogènes, des groupes alkyle en $C_1$–$C_4$, alcoxy en $C_1$–$C_4$;

- un reste alcényle en $C_3$–$C_{18}$ non substitué ou mono- à tétra-substitué par des halogènes ou monosubstitué par un groupe phényle ou méthoxycarbonyle;
- un reste alcynyle en $C_3$–$C_8$;
- un reste cycloalkyle en $C_3$–$C_{12}$ éventuellement substitué par des halogènes ou des groupes alkyle en $C_1$–$C_4$;
- un reste cycloalcényle en $C_3$–$C_8$;
- un reste phényle non substitué ou mono- ou polysubstitué par des halogènes, des groupes alkyle en $C_1$–$C_4$, alcoxy en $C_1$–$C_4$, alkylthio en $C_1$–$C_4$, $NO_2$, $CF_3$, COOH, CN, OH, $SO_3H$, $NH_2$ ou –NH(alkyle en $C_1$–$C_4$) ou –N(alkyle en $C_1$–$C_4$)$_2$;
- un noyau hétérocyclique à 5 ou 6 chaînons contenant 1 à 3 hétéroatomes;

$R_7$ et $R_8$ représentent chacun, indépendamment l'un de l'autre, l'hydrogène, un groupe alkyle en $C_1$–$C_6$ qui peut être interrompu par l'oxygène ou substitué par des halogènes, des groupes hydroxy ou cyano; un reste alcényle ou alcynyle en $C_2$–$C_6$ qui peut être substitué par des halogènes; un reste cycloalkyle en $C_3$–$C_6$; un reste phényle ou benzyle qui peut être substitué par des halogènes, des groupes alkyle en $C_1$–$C_4$, alcoxy ou alkylthio, nitro, cyano ou trihalogénométhyle, $R_7$ et $R_8$ peuvent également former avec l'atome d'azote sur lequel ils sont fixés un noyau hétérocyclique, X et Y représentent chacun un atome d'oxygène ou de soufre.

2. Les composés selon la revendication 1, répondant à la formule I dans laquelle Q représente le reste 2,4-dichlorophényle, 3,5-dichloropyrido-2-yle, 2-chloro-4-trifluorométhylphényle ou 3-chloro-5-trifluorométhylpyrido-2-yle, X et Y représentent chacun l'oxygène, n est égal à 0, $R_3$ représente un atome d'halogène, le groupe nitro ou le groupe cyano, $R_4$ représente l'hydrogène ou le groupe méthyle et A représente un groupe alcoxycarbonyle en $C_1$–$C_{18}$ ou alcoxyalcoxycarbonyle en $C_2$–$C_9$.

3. Les composés selon la revendication 1, de formule

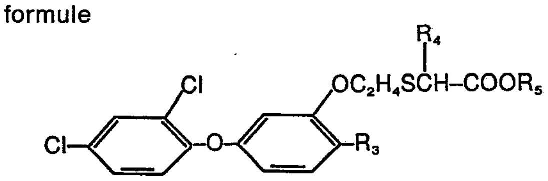

dans laquelle $R_3$ représente un atome d'halogène, un groupe nitro ou cyano, $R_4$ représente l'hydrogène ou un groupe méthyle, $R_5$ représente un groupe alkyle en $C_1$–$C_{18}$ ou alcoxyalkyle en $C_2$–$C_9$.

4. Les composés selon la revendication 1, répondant à la formule

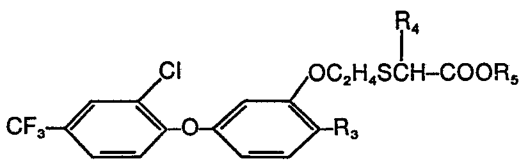

dans laquelle $R_3$ représente un atome d'halogène, le groupe nitro ou cyano, $R_4$ représente l'hydrogène ou un groupe méthyle, $R_5$ un groupe alkyle en $C_1$–$C_{18}$ ou alcoxy alkyle en $C_2$–$C_9$.

5. Les composés selon la revendication 1, de formule

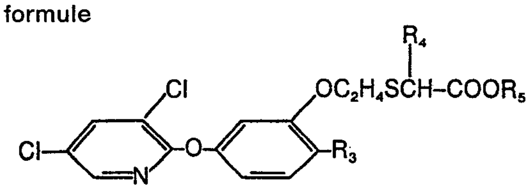

dans laquelle $R_3$ représente un atome d'halogène, un groupe nitro ou cyano, $R_4$ représente l'hydrogène ou un groupe méthyle, $R_5$ représente un groupe alkyle en $C_1$–$C_{18}$ ou alcoxyalkyle en $C_2$–$C_9$.

6. Les composés selon la revendication 1, de formule

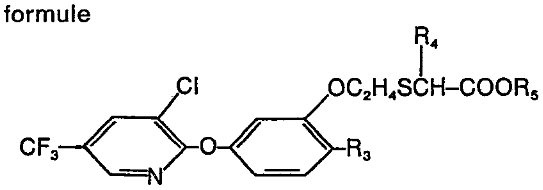

dans laquelle $R_3$ représente un atome d'halogène, le groupe nitro ou cyano, $R_4$ représente l'hydrogène ou un groupe méthyle, $R_5$ un groupe alkyle en $C_1$–$C_{18}$ ou alcoxyalkyle en $C_2$–$C_9$.

7. Le 2-[3-(2'-chloro-4'-trifluorométhylphénoxy)-6-chlorophénoxy-éthylthio]-propionate de méthyle selon la revendication 1.

8. Le 3-(2'-chloro-4'-trifluorométhylphénoxy)-6-chlorophénoxy-éthylthio-acétate de méthyle selon la revendication 1.

9. Le 3-(2'-chloro-4'-trifluorométhylphénoxy)-6-nitrophénoxy-éthylthio-acétate d'éthyle selon la revendication 1.

10. Le 2-[3-(2'-chloro-4'-trifluorométhylphénoxy)-6-nitrophénoxy-éthylthio]-propionate de méthyle selon la revendication 1.

11. Le 2-[3-(2'-chloro-4'-trifluorométhylphénoxy)-6-nitrophénoxy-éthylthio]-propionate d'éthyle selon la revendication 1.

12. Procédé de préparation des composés de formule I selon la revendication 1, caractérisé en ce que l'on fait réagir un phénol ou thiophénol de formule II

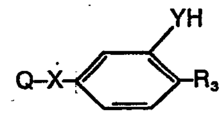
(II)

dans laquelle Q, $R_3$, X et Y ont les significations indiquées en référence à la formule I de la revendication 1, dans un solvant organique polaire, en présence d'une base en quantité suffisante pour fixer les acides, d'abord avec un 1,2-dihalogénoéthane de formule III

$$Hal-CH_2-CH_2-Hal \quad (III)$$

dans laquelle Hal représente un halogène, ce qui donne respectivement un halogénoéthoxybenzène ou un halogénoéthylthiobenzène qu'on fait réagir dans un solvant organique polaire, en présence d'une base en quantité suffisante pour neutraliser les acides, avec un composé de formule IV

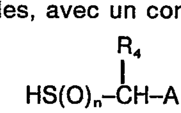
(IV)

dans laquelle A, n et $R_4$ ont les significations indiquées en référence à la formule I de la revendication 1.

13. Procédé de préparation des composés de formule I, caractérisé en ce que l'on fait réagir un composé de formule V

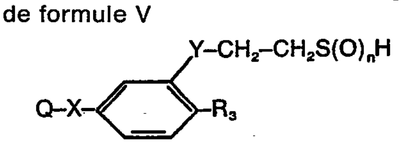
(V)

dans laquelle n, Q, $R_3$ et Y ont les significations indiquées en référence à la formule I de la revendication 1 dans un solvant organique polaire, en présence d'une base en quantité suffisante pour neutraliser les acides, avec un dérivé d'acide α-halogénocarboxylique de formule VI

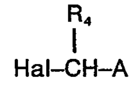

dans laquelle Hal représente un halogène, et A et $R_4$ ont les significations indiquées en référence à la formule I, de la revendication 1.

14. Procédé de préparation des composés de formule I, selon la revendication 1, caractérisé en

ce que l'on fait réagir un phénol ou thiophénol de formule II

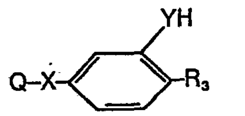

(II)

dans laquelle Q, $R_3$, X et Y ont les significations indiquées en référence à la formule I, de la revendication 1, dans un solvant polaire, en présence d'une base en quantité suffisante pour neutraliser les acides, avec un dérivé d'acide halogénoéthylthio, -sulfinyl ou -sulfonyl-alcane-carboxylique de formule VII

$$\mathrm{Hal{-}CH_2{-}CH_2{-}S(O)_n\overset{\overset{R_4}{|}}{C}H{-}A} \qquad (VIII)$$

dans laquelle Hal représente un halogène et A, n et $R_4$ ont les significations indiquées en référence à la formule I, de la revendication 1.

15. Procédé de préparation des composés sulfinylés et sulfonylés de formule I, caractérisé en ce que l'on oxyde un composé de formule Ia

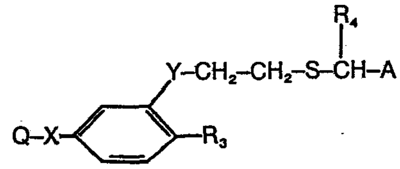

(Ia)

dans laquelle A, Q, $R_3$, $R_4$, X et Y ont les significations indiquées en référence à la formule I de la revendication 1, dans un solvant inerte, à l'aide d'un agent oxydant.

16. Produit herbicide et régulateur de la croissance des végétaux, caractérisé en ce qu'il contient en tant que composant actif au moins un composé de formule I, de la revendication 1.

17. L'utilisation des composés de la formule I selon la revendication 1 ou de produits en contenant pour la lutte contre les mauvaises herbes.

18. L'utilisation des composés de la formule I selon la revendication 1, ou de produits en contenant pour l'inhibition de la croissance des végétaux.

19. L'utilisation des composés de la formule I, selon la revendication 1, ou de produits en contenant, pour la dessiccation et la défoliation de végétaux cultivés peu avant leur récolte.